Europäisches Patentamt

European Patent Office

Office européen des brevets

(19)

(11) **EP 1 239 834 B1**

(12) **FASCICULE DE BREVET EUROPEEN**

(45) Date de publication et mention
de la délivrance du brevet:
**06.10.2004 Bulletin 2004/41**

(21) Numéro de dépôt: **00990090.3**

(22) Date de dépôt: **21.12.2000**

(51) Int Cl.[7]: **A61K 9/127**, A61K 9/133

(86) Numéro de dépôt international:
**PCT/FR2000/003631**

(87) Numéro de publication internationale:
**WO 2001/045672 (28.06.2001 Gazette 2001/26)**

(54) **STRUCTURES MIXTES RESULTANT DE L'INCORPORATION D'UNE MACROMOLECULE BIOLOGIQUE DANS UNE PHASE CRISTAL-LIQUIDE D'AMPHIPHILES**

GEMISCHTE STRUKTUREN BEI AUFNAHME VON BIOLOGISCHEN MAKROMOLEKÜLEN IN EINER FLÜSSIGKRISTALL-PHASE VON AMPHIPHILEN

MIXED STRUCTURES RESULTING FROM THE INCORPORATION OF A BIOLOGICAL MACROMOLECULE, ESPECIALLY DNA, IN A LIQUID CRYSTAL PHASE OF AMPHIPHILES, AND VESICLES OBTAINED USING THESE STRUCTURES

(84) Etats contractants désignés:
**AT BE CH CY DE DK ES FI FR GB GR IE IT LI LU MC NL PT SE TR**

(30) Priorité: **21.12.1999 FR 9916191**

(43) Date de publication de la demande:
**18.09.2002 Bulletin 2002/38**

(73) Titulaire: **CAPSULIS**
**33600 Pessac (FR)**

(72) Inventeurs:
• **POTT, Tanja**
**35000 Rennes (FR)**
• **LAVERSANNE, René**
**F-33600 Pessac (FR)**
• **ROUX, Didier**
**F-33700 Mérignac (FR)**

(74) Mandataire: **Giraud, Françoise et al**
**Cabinet Beau de Loménie**
**158, rue de l'Université**
**75340 Paris Cedex 07 (FR)**

(56) Documents cités:
**WO-A-95/09610         WO-A-97/07784**
**WO-A-98/02144         WO-A-98/23260**
**US-A- 5 679 355**

**Description**

La présente invention concerne de nouveaux produits résultant de l'incorporation de macromolécules biologiques, en particulier d'ADN, dans une phase cristal-liquide d'amphiphiles ainsi que les procédés d'obtention de ces produits.

La médecine traite jusqu'à présent une maladie en apportant une molécule exogène qui va corriger le dysfonctionnement en cause, ou au moins annuler ou limiter ses conséquences. Malheureusement l'emploi de la molécule active utilisée dans cette stratégie est la plupart du temps accompagnée d'effets secondaires indésirables. rendant la thérapie parfois inutilisable. D'autre part certaines maladies. liées à une déficience ou à un défaut génétique ne peuvent être traitées par une approche "chimique", soit parce que la molécule active n'existe pas. soit parce qu'elle est détruite avant son action. ou parce qu'il faudrait en administrer des doses trop importantes ou sur des durées trop longues.

La thérapie génique est une approche nouvelle pour aborder ces insuffisances de la médecine classique. Elle consiste à remplacer un gène déficient ou à apporter un nouveau gène permettant de soigner un état pathologique. Avec une telle stratégie. on n'apporte plus de médicament. mais on donne à l'organisme les moyens de lutter contre la maladie ou de corriger la déficience à l'origine de la maladie.

Cette stratégie, rendue possible par les progrès de la biologie moléculaire qui ont permis l'identification et la synthèse des gènes d'intérêt, nécessite de pouvoir apporter le gène utile jusqu'à sa cible cellulaire. puis de le faire pénétrer dans la cellule et enfin de le faire s'exprimer. Le gène doit être vectorisé. c'est-à-dire introduit dans un vecteur qui va lui faire passer les différentes barrières qui protègent l'organisme et la cellule de l'intrusion de matériel génétique étranger. Le vecteur idéal doit incorporer suffisamment d'ADN (ou d'ARN). le protéger des différentes attaques chimiques et enzymatiques rencontrées *in vivo*. être capable d'atteindre préférentiellement la cible cellulaire recherchée, pénétrer dans la cellule par exemple par endocytose. protéger l'ADN des conditions très contraignantes rencontrées dans l'endosome, apporter le gène thérapeutique jusqu'au noyau et le libérer en lui permettant de s'exprimer. ou mieux de s'intégrer au génome de la cellule.

Les virus sont des vecteurs d'ADN (ou d'ARN pour les rétrovirus) qui répondent parfaitement à la plupart de ces contraintes. Ils sont très étudiés comme vecteurs de gènes pour la thérapie génique, et font l'objet de nombreux essais cliniques. Ils ont cependant un défaut majeur, outre les difficultés de leur manipulation et de production à une échelle importante, c'est la suspicion qui pèse sur leur possibilité de devenir virulents et de provoquer des pathologies nouvelles incontrôlables.

Une importante recherche est consacrée à la mise au point de vecteurs synthétiques, ne présentant bien évidemment aucun risque de virulence. Les liposomes ont été très étudiés dans ce sens, mais ils souffrent de leurs défauts intrinsèques : très faible stabilité, grande difficulté de préparation, très faible capacité d'encapsulation et très faible, voire inexistante capacité à transfecter le gène. Une nouvelle classe de liposomes, préparés à partir de lipides cationiques a été développée, qui pallie partiellement les défauts des liposomes : meilleure stabilité, simplicité de préparation et taux de transfection significatif, bien que très faible par rapport aux virus. Cependant ces associations entre lipides cationiques et nucléotides ne sont pas des liposomes à strictement parler, ce sont plutôt des complexes entre le nucléotide, chargé négativement et le lipide chargé positivement (M. *Schmutz et al,* PNAS **96** p 12293 (1999))

La demande internationale WO 98/02144 enseigne qu'il est possible d'intégrer un nucléotide dans des vésicules multilamellaires à structure en oignon, de le protéger et de le transporter, permettant ainsi sa transfection intracellulaire.

Plus récemment, le brevet FR 2 766 706 a décrit des vésicules multilamellaires enroulées, à base de mélanges de lipides anioniques et cationiques, et leur application pour transfecter des nucléotides.

Des vecteurs d'administration de macromolécules sous forme de liposomes ou de vésicules sont également connus de WO 9 707 784, WO 9 509 610 et WO 9 823 260.

L'utilisation des liposomes pour vectoriser l'ADN ou les gènes se heurte, entre autres, à l'impossibilité d'introduire une quantité suffisante de nucléotides dans le liposome. Pour les liposomes classiques, aussi bien que pour les liposomes multilamellaires, ou bien les vésicules à structure en oignon, dont la taille est de l'ordre du micromètre, il est facilement concevable que la molécule d'ADN aura des difficultés à s'incorporer, sa taille étant du même ordre de grandeur. Il y a donc une nécessité à compacter la molécule, pour lui faire adopter une conformation repliée, qui lui donne une taille apparente compatible avec l'encapsulation dans ces vésicules lipidiques.

L'ADN étant chargé négativement, cette compaction est réalisée dans la nature par l'utilisation d'histones, protéines cationiques qui complexent l'ADN et conduisent à des particules de très petite taille (inférieure à 100 nm). De manière artificielle de telles molécules, ainsi que d'autres molécules cationiques ont été utilisées pour compacter l'ADN afin de lui permettre d'être incorporé dans des vésicules lipidiques de type liposomes ou vésicules multilamellaires.

Les travaux sur l'encapsulation de l'ADN par des liposomes à base de lipides neutres ont été complètement abandonnés à cause de leur faible pouvoir d'encapsulation (<5%). Les travaux actuels portent sur l'utilisation de molécules cationiques, soit de tensioactifs, soit de polymères, afin de former des complexes avec l'ADN susceptibles de protéger cette molécule et de permettre sa transfection, réalisant à la fois la compaction de la molécule d'ADN et sa vectorisation. Les particules ainsi obtenues sont souvent appelées liposomes cationiques, bien que leur mode de préparation et leur formulation soient assez différents des liposomes classiques.

Des travaux très récents (cf., par exemple, I Koltover, *et al*, Science, **281**, p78 1998 ; J.O. Räder, *et al*, Science, **275** p 810 1997 : T. Salditt *et al*. Physical Review Letters, **79** p 2582 1997) ont démontré que, dans ces complexes, l'ADN formait des phases de symétrie cristal-liquide, venant s'intercaler avec la phase cristal-liquide formée par les tensioactifs cationiques.

Plusieurs symétries ont été identifiées, et les expériences de diffraction des rayons X (synchrotron) montrent clairement la superposition des diagrammes de diffraction de chacun des systèmes de tensioactifs et d'ADN. Plusieurs lipides cationiques ont été utilisés, conduisant à des symétries différentes de la phase cristal-liquide des lipides. Les travaux cités ont mis en évidence l'importance du rôle de la charge des lipides, ainsi que de celui de la charge totale du complexe, qui peut être positive ou négative suivant le rapport entre lipides cationiques et ADN négatif. De plus, il est suggéré que ces structures de phases cristal-liquide intercalées jouent un rôle important dans les résultats obtenus en transfection.

Les amphiphiles, communément appelés tensioactifs, ont la capacité, lorsqu'ils sont introduits dans un solvant, par exemple l'eau, de s'auto-associer pour former des phases structurées ou organisées. Parmi ces phases, on distingue en particulier les phases cristal-liquide, thermodynamiquement stables, qui présentent un ordre cristallographique partiel (dans une ou deux directions de l'espace) comme les phases lamellaires (ou smectiques) qui ont un ordre unidimensionnel, ou les phases hexagonales présentant un ordre bidimensionnel. Il existe aussi des phases présentant un ordre cristallin comme la phase cubique. Ces phases sont souvent dites lyotropes car leur apparition et leur stabilité dépendent de la concentration de l'amphiphile dans le solvant. Elles peuvent être aussi thermotropes si leur domaine d'existence dépend de la température. Une description de ces phases peut être trouvée dans C.L. Khetrapol *et al*, Nuclear magnetic résonance studies in lyotropic liquid-crystals, 1975. Dans la suite nous utiliserons le terme phase structurée pour décrire d'une manière générale ces phases, sauf dans les cas où la symétrie de la phase est explicitement indiquée.

Les inventeurs de la présente invention ont maintenant découvert, qu'il était possible d'obtenir des structures de phases cristal-liquide intercalées d'ADN et de tensioactifs, sans avoir besoin d'utiliser des lipides cationiques. Contrairement à ce qui était indiqué dans la littérature, et de façon tout à fait surprenante, le mélange d'une phase cristal-liquide de tensioactif neutre, par exemple zwitterionique, par exemple d'une phase lamellaire, avec de l'ADN peut conduire sous certaines conditions à la formation d'une phase mixte, dans laquelle les tensioactifs forment des feuillets lamellaires empilés selon une symétrie smectique, au sein desquels sont intercalées les molécules d'ADN, elles-mêmes arrangées selon une symétrie cristal-liquide de type nématique. Qui plus est, et de manière encore plus surprenante, ces résultats sont obtenus uniquement lorsqu'on introduit une forte quantité d'ADN dans la phase lamellaire, alors même qu'il est difficile d'introduire avec un bon rendement une faible quantité d'ADN dans la phase lamellaire.

Poursuivant leurs recherches, les inventeurs de la présente invention ont pu établir qu'il était de la même façon possible d'introduire différentes macromolécules biologiques, en particulier des macromolécules nucléotidiques, des protéines ou des polysaccharides dans des phases structurées, et, cela, sans interaction de type électrostatique entre lesdites macromolécules et les composants de la phase structurée, à condition toutefois d'introduire une quantité suffisante de ces macromolécules au sein de cette phase structurée.

Parmi les macromolécules nucléotidiques constituées d'enchaînement de nucléotides et très souvent désignées sous le vocable générique "nucléotide", on distingue, selon la présente invention, les oligonucléotides comprenant généralement de 10 à 30 groupements nucléotidiques de base et au maximum 100, et les polynucléotides comprenant un plus grand nombre de groupements nucléotidiques de base, généralement plus de 100 paires de base, et souvent plus de 1.000. Même si elle n'exclut pas le cas des oligonucléotides tels que définis précédemment, l'invention s'applique tout particulièrement aux macromolécules de type polynucléotides tels que définis ci-dessus et, en particulier à l'ADN.

Ainsi donc, les inventeurs ont pu étendre les résultats concernant l'introduction de l'ADN en phase concentrée dans une phase structurée à d'autres polymères biologiques.

L'invention a ainsi rendu possible l'incorporation de macromolécules dans une phase structurée formée de membranes de tensioactifs. alors même que ces macromolécules présentent une taille caractéristique supérieure à l'espace entre les lamelles amphiphiles de cette phase de tensioactifs. La macromolécule se trouve introduite sous une forme concentrée dans la nouvelle structure de l'invention et se trouve donc à une forte concentration dans le système final formé.

Ainsi, l'invention conduit à un mélange structuré, ci-après dénommé phase mixte structurée, formé par l'incorporation dans une phase cristal-liquide d'amphiphiles, de macromolécules dont la taille caractéristique est supérieure à celle de la phase cristal-liquide et dont le rapport massique par rapport aux amphiphiles est supérieur à 5 %. sans l'intervention d'interaction électrostatique entre les macromolécules et les amphiphiles

Il est toujours possible de définir une taille caractéristique de la macromolécule, mais celle-ci dépend de la nature de cette macromolécule. D'une manière générale, on peut citer le rayon de giration. mesuré par exemple à partir d'expériences de diffusion statique de la lumière. Plus précisément dans le cas des protéines, on prendra le rayon de giration de la protéine non dénaturée. la dénaturation s'accompagnant généralement d'une variation de ce rayon. Dans le cas de l'ADN, il s'agit de la longueur de la molécule non condensée. et elle est simplement obtenue à partir de la longueur caractéristique d'une paire de base (3.4 A), en multipliant par le nombre de paires de base.

On peut aussi toujours définir une longueur caractéristique pour la phase cristal-liquide. D'une manière générale il s'agira de la taille de la maille cristalline, mono- ou bidimensionnelle. Elle est apparentée au vecteur d'onde des pics de diffraction des rayons X. Plus concrètement. et à titre d'exemples. dans le cas de la phase lamellaire. ce sera la périodicité d'empilement des lamelles, dans le cas de la phase hexagonale. il s'agira de la distance entre tubes de tensioactifs.

Le principal intérêt de l'invention est qu'elle fournit un moyen pour former des structures concentrées en macromolécules biologiques sans recourir. comme cela est connu dans l'art antérieur, à une incorporation dans laquelle la force motrice est de nature électrostatique. L'invention permet donc de s'affranchir des charges électriques qui. dans l'art antérieur. viennent perturber l'activité biologique du complexe formé en provoquant, par exemple, l'adhésion de ces complexes aux parois des cellules et leur capture par des protéines plasmatiques.

Par ailleurs, comme cela ressort de la description qui suit, l'invention permet également de préparer à partir de ces structures mixtes des vésicules multilamellaires incorporant lesdites macromolécules avec des taux d'cncapsulation à la fois élevés et contrôlables.

Ainsi, selon l'une de ses caractéristiques essentielles, l'invention concerne une phase mixte structurée formée par l'incorporation d'une macromolécule biologique dans une phase cristal-liquide à base de composés amphiphiles dans laquelle ladite macromolécule biologique est présente dans ladite phase mixte structurée à raison d'au moins 5 % en poids par rapport aux composés amphiphiles et l'une au moins des deux entités constituées par la macromolécule biologique d'une part, et l'ensemble des composés amphiphiles d'autre part, présente un caractère non chargé.

Selon une variante particulièrement avantageuse de l'invention, la phase cristal-liquide d'amphiphiles est une phase cristal-liquide lamellaire.

Les phases mixtes structurées de la présente invention sont des mélanges dans lesquels la macromolécule vient s'intercaler sous une forme ordonnée à longue distance, dans une phase d'amphiphiles, elle-même ordonnée à longue distance, de type cristal-liquide.

Par phase cristal-liquide, on entend une phase cristal-liquide obtenue par mélange d'amphiphiles. Une telle phase peut généralement être caractérisée par sa composition, d'une part, et par une dimension caractéristique, par exemple son pas smectique qui est la distance entre les lamelles de ladite phase dans le cas de la phase lamellaire d'autre part.

Par amphiphile non chargé, on entend une molécule présentant un caractère amphiphile, mais dont la charge globale est neutre. Cela comprend les molécules non-ioniques, mais aussi les molécules amphotères dans lesquelles les charges positives et négatives se compensent au sein de la même structure chimique, et non pas grâce à un contre-ion mobile.

Comme exposé précédemment, il est en effet tout à fait surprenant d'avoir réussi à incorporer au sein d'une telle phase des macromolécules biologiques présentant des dimensions caractéristiques bien supérieures à celles de la phase cristal-liquide et, cela, sans faire appel à des interactions électrostatiques.

En effet, l'invention a permis d'introduire des quantités importantes d'ADN, ou plus généralement de macromolécules nucléotidiques ou de macromolécules biologiques dans des phases cristal-liquide, à base de tensioactifs non chargés, c'est-à-dire dans des conditions où, contrairement à ce qui a été fait dans l'art antérieur, il n'y a aucune interaction de type électrostatique entre les tensioactifs et les macromolécules.

Il a également été possible, dans le cadre de l'invention, d'introduire des macromolécules non chargées dans des phases cristal-liquide à base de tensioactifs, donc là aussi, sans qu'il y ait d'interaction électrostatique permettant d'expliquer une telle introduction et cela, alors que lesdites macromolécules sont nettement plus "grosses" que la distance entre les lamelles de la phase cristal-liquide et, sans déformation notable de ladite phase.

Les macromolécules biologiques visées par la présente invention sont essentiellement, comme exposé précédemment, des enchaînements de nucléotides, et en particulier des enchaînements de type "polynucléotides" tels que définis précédemment, des protéines, des polysaccharides.

Les polynucléotides sont des macromolécules formées par la succession des bases constitutives du code génétique. On distingue principalement l'ADN et l'ARN. Ce sont des macromolécules de très grande taille, dont la longueur peut atteindre 10 µm. Du fait de leur structure chimique, les polynucléotides portent toujours des charges négatives, contrebalancées par des cations.

Les protéines sont des macromolécules formées par l'assemblage d'acides aminés. Leur masse molaire peut aller jusqu'à plus de 200 000 Da, avec des rayons de giration pouvant atteindre plusieurs centaines d'Angström. Les protéines peuvent être cationiques, neutres ou anioniques selon la nature et la proportion des différents acides aminés qui les composent, mais aussi en fonction du pH, les fonctions acides et amines se protonant et déprotonant en fonction du pH. Cependant la majorité des protéines, dans des conditions normales de pH (voisines de la neutralité), sont anioniques.

Les polysaccharides sont formés de chaînes saccharidiques, allant de quelques unités sucre à plusieurs millions. Les tailles peuvent ainsi être extrêmement variées et atteindre des dimensions quasi macroscopiques. Suivant la nature des fonctions chimiques substituant les cycles saccharidiques, et en fonction du pH, les polysaccharides peuvent présenter différentes charges électriques.

Même si l'invention présente un intérêt tout particulier dans le cas des macromolécules biologiques dont le rayon de giration est très nettement supérieur à la distance entre les lamelles amphiphiles de la phase cristal-liquide, elle n'est toutefois pas limitée à de telles macromolécules.

Tout tensioactif susceptible, en mélange par exemple avec de l'eau, de former une phase cristal-liquide peut être utilisé dans l'invention. Dans les applications biologiques préférées, on prendra soin d'utiliser des amphiphiles compatibles avec l'application, et en particulier faiblement toxiques et bien tolérés, en particulier au niveau cellulaire. L'invention n'est pas liée à un tensioactif particulier ou à une famille particulière de tensioactifs, mais à une propriété intrinsèque des phases organisées de tensioactifs. Les amphiphiles utilisables sont avantageusement choisis dans le groupe constitué par :

- des glycérolipides, en particulier des glycérolipides phospholipidiques, hydrogénés ou non
- des acides gras en $C_6$ à $C_{30}$, saturés ou mono- ou polyinsaturés, linéaires ou ramifiés, éthoxylés ou non, sous forme d'acide ou de sel d'un métal alcalin, alcalino-terreux ou d'une amine,
- des esters éthoxylés ou non, de saccharose, ou de sorbitol, ou de mannitol, ou de glycérol ou de polyglycérol contenant de 2 à 20 motifs glycérol ou de glycol de ces mêmes acides gras,
- des mono-, di- ou triglycérides ou des mélanges de glycérides de ces mêmes acides gras,
- des alcools gras en $C_6$ à $C_{30}$, saturés ou mono- ou polyinsaturés, linéaires ou ramifiés, éthoxylés ou non,
- du cholestérol et ses dérivés, en particulier les esters de cholestérol chargés ou neutres comme le sulfate de cholestérol,
- des autres dérivés à squelette stérol, en particulier ceux d'origine végétale tels que le sitostérol ou le sigmastérol,
- des éthers, éthoxylés ou non, de saccharose, ou de sorbitol, ou de mannitol, ou de glycérol ou de polyglycérol contenant de 2 à 20 motifs glycérol ou de glycol et de ces mêmes alcools gras,
- des huiles végétales polyéthoxylées, hydrogénées ou non hydrogénées,
- des polymères séquencés de polyoxyéthylène et de polyoxypropylène (poloxamères),
- de l'hydroxystéarate de polyéthylèneglycol,
- des sphingolipides, et des dérivés de la sphingosine,
- des polyalkylglucosides,
- des céramides,
- des copolymères de polyéthylèneglycol et d'alkylglycol, par exemple, les copolymères de la famille des ELFACOS de AKZO NOBEL.
- des copolymères di- ou tribloc d'éthers de polyéthylèneglycol et de polyalkylèneglycol, par exemple les copolymères de la famille des ARLACELL de ICI.

Ces amphiphiles ou tensioactifs peuvent être utilisés seuls ou en mélange. Parmi ces amphiphiles. certains peuvent à eux seuls former une phase cristal-liquide. par exemple lamellaire. par mélange avec un solvant polaire. D'autres sont utilisés uniquement en mélange, en plus faible proportion, pour apporter des propriétés de rigidité ou d'élasticité à la phase cristal-liquide.

L'homme de l'art saura choisir les molécules les plus à même de former une phase cristal-liquide parmi ces molécules et contrôler la formation de ladite phase cristal-liquide par microscopie optique. On remarquera que la plupart des tensioactifs cités ci-dessus sont non chargés et ne sont donc pas susceptibles d'intervenir par une interaction électrostatique dans la condensation de la macromolécule que l'on souhaite insérer dans la phase cristal-liquide. De tels tensioactifs non chargés sont donc adaptés à l'incorporation de macromolécules chargées telles que l'ADN. Dans la liste donnée ci-dessus. certains tensioactifs présentent une charge, c'est le cas en particulier des sels d'acides gras ou de certains phospholipides ou des dérivés de la sphingosine qui pourront être utilisés pour l'incorporation de macromolécules non chargées dans les phases mixtes de l'invention.

Comme exposé précédemment, une des caractéristiques essentielles de l'invention est qu'elle permet de faire des structures concentrées en macromolécules sans avoir recours à des forces électrostatiques. Ainsi, lorsque la macromolécule présente un caractère ionique, on utilisera selon l'invention des agents amphiphiles non chargés. Toutefois, on pourra également admettre selon l'invention d'introduire dans les compositions de l'invention des quantités relativement faibles de produits à caractère ionique pour conférer, par exemple. des propriétés particulières à la structure mixte de l'invention ou aux vésicules obtenues par dispersion de cette structure mixte (comme cela ressort de la description qui suit). Dans ce cas. on distinguera le tensioactif principal qui n'a pas besoin de présenter une quelconque ionicité. de l'additif éventuellement ionique ajouté afin de modifier les propriétés, par exemple les propriétés d'adhésion aux parois cellulaires. de la structure formée. Cette distinction est facile à réaliser puisque la structure originale de l'invention, obtenue par incorporation d'une grande quantité de macromolécule biologique au sein d'une phase cristal liquide. peut être obtenue en absence de l'additif. mais évidemment pas en l'absence du tensioactif principal.

Il est donc important de noter que, plus que la notion de quantité relative entre le tensioactif principal et l'additif, c'est le rôle respectif de chacun qui les distingue. L'additif, qui pourra être lui-même un tensioactif ou bien un polymère par exemple, pourra être ionique. Dans ce cas. il pourra être soit inclus dans la structure. soit greffé ou adsorbé à la surface. L'additif pourra ainsi apporter par exemple une propriété d'adhésion de surface, intéressante pour favoriser la capture du mélange incluant la macromolécule par une cellule.

Le mode de préparation préféré de ces nouvelles structures est très similaire à celui des vésicules multilamellaires de tensioactifs.

D'une façon générale, ce procédé comprend les étapes de :

- préparation d'un mélange homogène contenant les composés amphiphiles et la macromolécule. ladite macromolécule étant présente dans des proportions pondérales d'au moins 5 % par rapport auxdits composés amphiphiles.
- d'incorporation d'une quantité adéquate de milieu aqueux ou polaire pour former une phase cristal-liquide avec lesdits composés amphiphiles.

La quantité adéquate de milieu aqueux pourra être contrôlée par microscopie optique ou polaire permettant de mettre en évidence la formation de la phase cristal-liquide.

La valeur de 5 % donnée ci-dessus est une valeur minimale pour obtenir la phase mixte structurée de l'invention. L'homme du métier comprendra aisément que les valeurs optimales de ce rapport varient avec la nature de la macromolécule et ses dimensions ainsi qu'avec la nature des agents amphiphiles utilisés. D'une manière générale, on a constaté que l'incorporation de la macromolécule dans la phase cristal-liquide se faisait d'autant mieux que la macromolécule était amenée en plus grande quantité. Les valeurs limites que l'on peut rencontrer. sont plus liées à des considérations d'ordre pratique, en particulier les concentrations maximales de macromolécule qui conduisent à des solutions ou dispersions assez fluides pour être manipulées, qu'à des considérations théoriques. On peut toutefois aisément imaginer qu'un rapport 1/1 entre tensioactif et macromolécule est une limite raisonnable. si elle n'est pas absolue.

Ainsi. dans le cas de la préparation d'une phase mixte structurée selon l'invention contenant une macromolécule constituée d'un enchainement de groupements nucléotidiques, en particulier de l'ADN, le mode de préparation préféré est très similaire à celui des vésicules multilamellaires de tensioactifs. Il consiste à préparer une phase lamellaire à partir d'un tensioactif ou d'un mélange de tensioactifs et d'une phase aqueuse. La phase aqueuse contient au moins un soluté assez concentré d'une telle macromolécule, en particulier d'une molécule d'ADN ou d'ARN.

Le rapport p en masse entre amphiphiles et macromolécules nucléotidiques est compris entre 20 et 1, de préférence entre 5 et 1 (ce qui signifie que pour 1 g de tensioactifs, on a de 0.2 g à 1 g de macromolécule nucléotidique).

Le mélange pour préparer la phase lamellaire peut se faire en une seule étape, tous les composants étant pesés séparément et additionnés ensemble. Dans ce cas on peut de manière optionnelle, afin de favoriser l'homogénéité du mélange procéder à un ou plusieurs cycles de congélation/décongélation, et/ou un ou plusieurs cycles de lyophilisation et réhydratation.

Si certains composants du mélange sont difficiles à solubiliser (par exempte cholestérol), on peut aussi éventuellement utiliser un solvant, par exemple un alcool ou un solvant chloré, ou un mélange de ces solvants afin de dissoudre ces composants, puis après évaporation, obtenir un mélange homogène de ces composants. qui donnera par hydratation la phase cristal-liquide. de préférence lamellaire.

La méthode exacte de préparation n'est pas critique pour la réussite de l'incorporation à condition que la phase cristal-liquide, de préférence lamellaire, soit concentrée et qu'il n'y ait pas d'excès de solvant (phase aqueuse), et certaines méthodes classiques utilisées pour augmenter le taux d'encapsulation d'un produit dans les liposomes ou autres vésicules multilamellaires de phospholipides ou autres amphiphiles non chargés peuvent très bien être utilisées. On veillera toutefois à éviter tout excès d'eau ou de solvant aqueux ou polaire qui pourrait conduire directement à la formation de vésicules de type liposome classique. La quantité d'eau ou de milieu aqueux ou de solvant polaire devra en effet, comme indiqué précédemment. correspondre à la quantité adéquate pour former une phase cristal-liquide. en particulier une phase cristal-liquide lamellaire.

Les essais réalisés par les inventeurs de la présente invention ont permis de caractériser clairement les nouvelles structures formées et, cela en s'appuyant sur des résultats obtenus en diffraction des rayons X des phases obtenues par mélange de tensioactifs et de macromolécules à différentes concentrations. traçant ainsi le diagramme de phase du système eau/tensioactif/macromolécule ainsi que sur l'analyse de la densité électronique déduite des intensités des pics de diffraction.

**EP 1 239 834 B1**

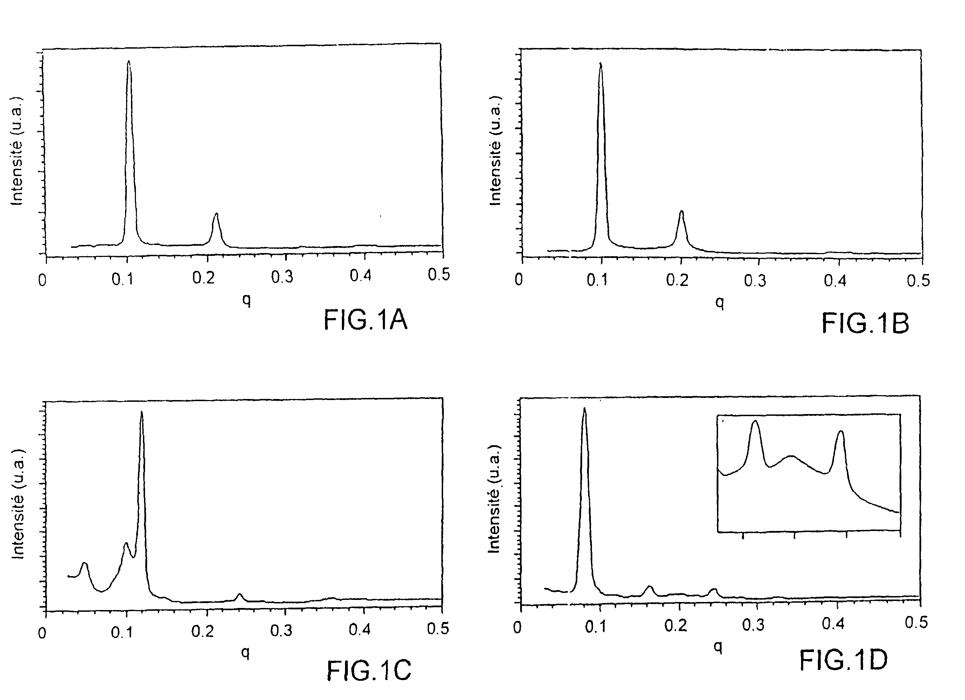

Les différents résultats obtenus ressortant de la description et des exemples qui suivent sont donnés également en référence aux figures 1 à 5 annexées qui représentent respectivement

Figures 1A à 1D : L'intensité de diffraction de rayons X (synchrotron) des phases lamellaires à 50% d'eau en fonction du vecteur d'onde q (q=4$\pi$ sin$\theta$/$\lambda$) pour différents rapports massiques lipide/ADN, p.

- Figure 1A : $\rho = \infty$ (absence d'ADN),
- Figure 1B : $\rho = 80$,
- Figure 1C : $\rho = 20$, et
- Figure 1D : $\rho = 2$. Sur la figure 1D, le pic de corrélation entre brins d'ADN est indiqué par une flèche dans l'insert.

Figure 2A : Le profil de densité électronique le long de l'axe d'empilement des couches de lipides pour la phase lamellaire à $\rho = 2$ et 47.4% $H_2O$ (ligne ininterrompue). Pour comparaison le profil de densité électronique d'une phase lamellaire en absence d'ADN est également montré (pointillé).

Figure 2B : Une représentation schématique de la phase lamellaire contenant l'ADN organisé ($L_{\alpha}^c$); la flèche longue horizontale indique la distance de répétition de la phase lamellaire (autour de 80Å), la flèche courte verticale indique la distance entre brins d'ADN (30 à 40Å).

Figure 3 : Le taux d'encapsulation (mesuré comme la quantité d'ADN encapsule à l'intérieur. par rapport à la quantité totale d'ADN incorporé dans la formulation) des vésicules obtenues à partir d'une phase $L_{\alpha}^c$ avec $\rho = 2$, porté en fonction de l'osmolarité (exprimée en milliosmomol) du milieu de dispersion.

Figure 4 : Le profil de diffraction synchrotron de rayons X des vésicules obtenues à partir de la phase $L_{\alpha}^c$ à $\rho = 2$ et 50% $H_2O$, dispersées dans un milieux à 603 (ligne ininterrompue) et 1457 (pointillé) mOsm. Les flèches dans l'insert indiquent les pics de corrélation entre brins d'ADN.

Figure 5 : Le rapport massique lipide/ADN encapsulé, $\rho^*$, des vésicules contenant de l'ADN à $\cong$150 pb (gris foncé) ou un plasmide à 5581 pb (gris clair) déterminé après dilution des vésicules dans un tampon PBS à 300 mOsm en fonction du rapport massique lipide/ADN, $\rho$, de la phase lamellaire.

On donne ci-après les résultats observés dans le cas de l'ADN. Cette molécule a été choisie pour deux raisons. la première parce qu'il existait une véritable impossibilité d'encapsuler dans des microvésicules non cationiques des quantités utiles d'ADN. La seconde parce que d'un point de vue démonstratif, l'ADN, de par sa très grande longueur et sa structure particulière est certainement le défi le plus difficile à relever. L'homme de l'art comprendra que si l'on peut par cette méthode encapsuler de grandes quantités d'ADN, molécule particulièrement encombrante. la généralisation aux autres macromolécules comme les protéines ou les polysaccharides dont le comportement est plus commun ne présente pas de difficultés majeures.

La principale preuve expérimental de l'incorporation de la macromolécule nucléotidique dans la phase lamellaire est obtenue par diffraction des rayons X. En effet, la phase lamellaire, comme toute phase cristal-liquide. présentant une période de répétition le long de l'axe d'empilement. va donner un signal de diffraction en rayons X, appelé pic de Bragg. La position de ce pic de diffraction (exprimée en angle de diffraction. ou en vecteur d'onde q) dépend directement de la distance de répétition. De plus les intensités des pics sont reliées au profil de densité électronique du motif répétitif.

Les observations en diffraction des rayons X ont été effectuées pour différentes valeurs du rapport massique lipides/ADN, $\rho$, et de concentration en eau des échantillons. Quatre graphes de diffraction à hydratation constante (50% d'eau) sont donnés (voir figures 1A à 1D). en absence d'ADN, pour $\rho = 80$ (faible charge en ADN) , $\rho = 20$ (charge en ADN moyenne) et pour $\rho = 2$ (forte charge en ADN).

Pour le système en absence d'ADN ou pour des faibles quantités d'ADN on observe des pics de Bragg caractéristiques d'une phase iamellaire (voir figures 1A et 1B). La distance de répétition à $\rho = 80$ ($\cong$59Å) est légèrement réduite par rapport au système en absence d'ADN ($\cong$63Å), probablement à cause d'une déshydratation partielle de la phase lamellaire due à la présence d'ADN non incorporé.

Pour $\rho = 20$, on observe la coexistence de deux systèmes de diffraction, révélatrice de la coexistence de deux phases lamellaires (voir figure 1C), l'une dépourvue d'ADN avec une distance de répétition plus faible ($\cong$ 53Å) que la phase lamellaire en absence d'ADN. l'autre enrichie en ADN montrant une très forte augmentation de distance de répétition ($\cong$129Å).

Pour $\rho = 2$, on observe l'existence d'un seul système lamellaire. avec une distance de répétition augmentée ($\cong$77Å) par rapport à la phase lamellaire ne contenant pas l'ADN (voir figure 1D). De plus, on observe un pic de corrélation entre brins d'ADN ($\cong$32Å). Ce pic se déplace lors du changement de la quantité d'ADN Jans la phase lamellaire, démontrant que l'ADN est effectivement incorpore dans la phase lamellaire de lipides, sous une forme ordonnée. De plus il a ete observé que. en faisant varier la concentration en eau d'un tel système, les deux distances de répétition, celle de la phase lamellaire, et celle de la distance de correlation entre molécules d'ADN variaient de la même manière. On en déduit l'existence, surprenante, à haute concentration en ADN (le phénomène est observable pour $\rho < 6$) d'un réseau d'ADN intercalé entre les couches du réseau de lipides.

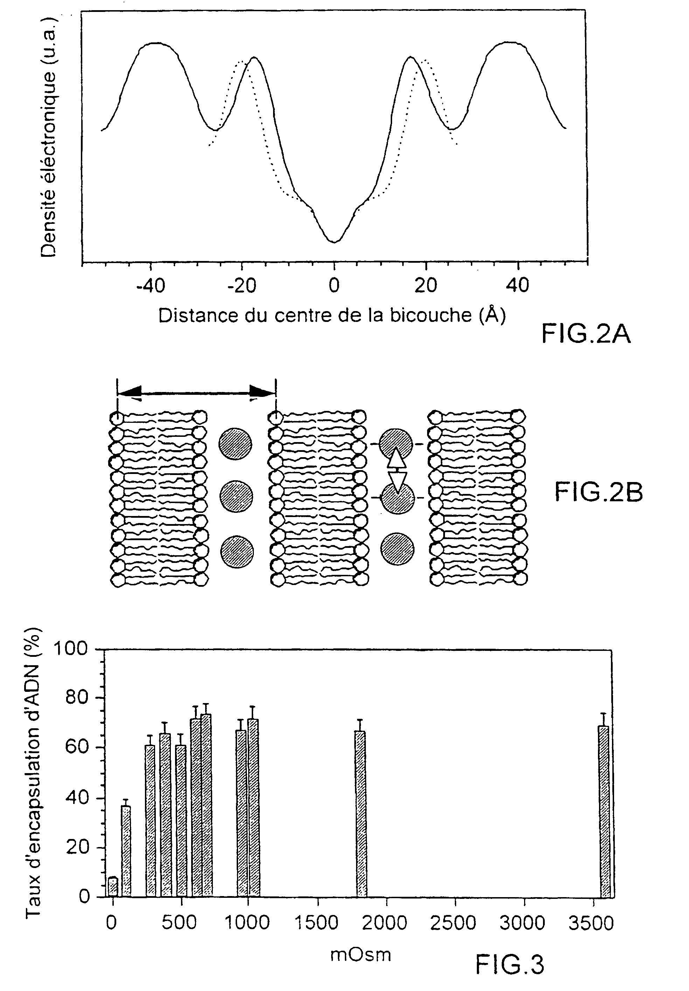

Une autre preuve de l'existence de ce système mixte de réseaux intercalés de phase lamellaire de lipide et de phase organisée d'ADN est déduite de l'analyse de la densité électronique, à partir des intensités des pics de diffraction. Le profil de densité électronique d'une phase lamellaire lipide/ADN/eau ($\rho$ = 2) le long de l'axe d'empilement des couches de lipides est reporté sur la figure 2. Pour comparaison un profil de densité électronique d'une phase lamellaire en absence d'ADN est également montré en pointillé sur la figure. La différence majeure entre ces deux profils est la présence d'une bosse de forte densité électronique à environ 40A du centre de la bicouche lipidique et d'une largeur à mi-hauteur voisine de 10Å. Ceci est compatible avec la présence de molécules d'ADN intercalées dans la phase aqueuse entre les bicouches de lipides. Dans la suite cette phase lamellaire contenant l'ADN organisé sera appelée $L_\alpha^c$.

Ces observations sont tout à fait similaires à celles qui avaient été faites avec les lipides cationiques (cf. I Koltover, *et al*. Science. **281**. p78 1998 : J.O. Räder, *et al*. Science. **275** p 810 1997 : T. Salditt *et al*. Physical Review Letters. 79 p 2582 1997). mais de façon tout à fait inattendue. ce phénomène est obtenu pour des phases de tensioactifs non chargés.

Ce résultat est surprenant car les explications avancées par les auteurs des travaux sur l'incorporation d'ADN dans des phases lamellaires de lipides cationiques étaient liées à la présence des fortes interactions ioniques entre les lipides et le nucléotide. Dans le cas de l'invention, de telles interactions n'existent pas. et l'explication connue de l'homme de l'art n'est donc pas valable. Sans que cela soit une preuve, ni une explication du phénomène observé, on peut avancer que dans le cas de l'invention, la stabilité thermodynamique de l'édifice obtenu (réseau mixte de lipides et d'ADN) uniquement à forte concentration d'ADN pourrait être liée à la faible perte d'entropie d'un tel système où les deux sous-ensembles sont ordonnés, par rapport à un système moins concentré, où ce terme entropique ne serait pas aussi efficace.

Les nouvelles structures mises en évidence dans le cadre de la présente invention décrites ci-dessus s'avèrent particulièrement intéressantes du fait de la possibilité de transformer ces nouvelles structures, par dispersion, en vésicules multilamellaires à structure en oignon incorporant en leur sein lesdites macromolécules à des concentrations jamais observées à ce jour.

Ainsi. selon un autre de ses aspects. l'invention concerne des vésicules obtenues à partir des milieux mixtes de l'invention, par simple dispersion de ce milieu ainsi qu'un procédé pour préparer ces vésicules et, en particulier pour jouer sur le taux d'encapsulation des macromolécules au sein desdites vésicules.

Les essais réalisés par les inventeurs de la présente invention ont en effet permis de mettre clairement en évidence la présence effective des macromolécules dans la phase lamellaire même après dispersion de celle-ci en milieu aqueux. c'est-à-dire dans des conditions où la phase $L_\alpha^c$ est thermodynamiquement instable.

Par ailleurs, les essais réalisés par les inventeurs de la présente invention ont permis de montrer comment. en jouant sur la force ionique du milieu de dispersion. on pouvait jouer sur le taux d'encapsulation de ladite macromolécule au sein desdites vésicules.

On donne ci-dessous des résultats obtenus avec l'ADN. Comme indiqué précédemment, la généralisation aux autres macromolécules biologiques ne présente pas de difficultés, le comportement de ces macromolécules étant toujours plus simple que celui de l'ADN. Les considérations d'influence de la force ionique sur la variation du taux d'encapsulation sont directement transposables à ces macromolécules, le phénomène étant plus lié aux pressions osmotiques engendrées par les différences de force ionique et à leurs conséquences sur l'intégrité des vésicules. qu'à l'influence de celle-ci sur la conformation de la macromolécule.

Des expériences ont aussi été réalisées pour vérifier la présence effective de l'ADN dans la phase lamellaire. même après dispersion de celle-ci en milieu aqueux, c'est à dire dans des conditions où la phase $L_\alpha^c$ est thermodynamiquement instable. Il a été montré que, compte tenu de la très forte concentration en ADN dans la phase lamellaire de lipide, entraînant une osmolarité importante, il est indispensable de disperser la phase lamellaire contenant l'ADN dans un milieu aqueux de force ionique suffisante. Cela a été obtenu par utilisation d'un tampon aqueux comme milieu de dispersion. Le résultat est donné sur la courbe représentée sur la figure 3, où le rendement d'encapsulation (mesuré comme la quantité d'ADN encapsulée à l'intérieur. par rapport à la quantité totale d'ADN incorporée dans la formulation) est porté en fonction de l'osmolarité du milieu de dispersion. Dans la suite, l'unité utilisée pour l'osmolarité est le milliosmomol (mOsm), unité couramment utilisée dans le domaine biologique. Cette unité est reliée au pascal par une relation linéaire : 100 mOsm = 2.4777 $10^5$ Pa.

On observe que, pour une osmolarité supérieure à $\cong$500 milliosmomol, le rendement d'encapsulation est constant autour de 70%, mais on notera également qu'un bon taux d'encapsulation d'environ 60% est déjà atteint à 300 mOsm (milieu physiologique).

De plus, nous avons démontré un phénomène surprenant. Il est possible de disperser les vésicules de l'invention à une osmolarité supérieure à celle du milieu physiologique afin d'optimiser le taux d'encapsulation d'ADN et de les diluer ensuite dans un milieu physiologique sans perte d'ADN encapsulé. Nous avons de plus démontré que l'ADN encapsulé dans les vésicules selon l'invention à base des lipides neutres est en effet protégé contre sa dégradation par la DNase et que ces dispersions ont une très bonne stabilité (diminution du taux d'encapsulation d'environ 70% à environ 60% après un mois).

Plus généralement, les dispersions de vésicules de l'invention peuvent être obtenues dans un procédé comprenant les étapes de :

- dispersion dans un milieu de même osmolarité que le milieu interne,
- modification de l'osmolarité du milieu externe par ajout de sel ou d'eau ou par dialyse afin d'atteindre l'osmolarité souhaitée.

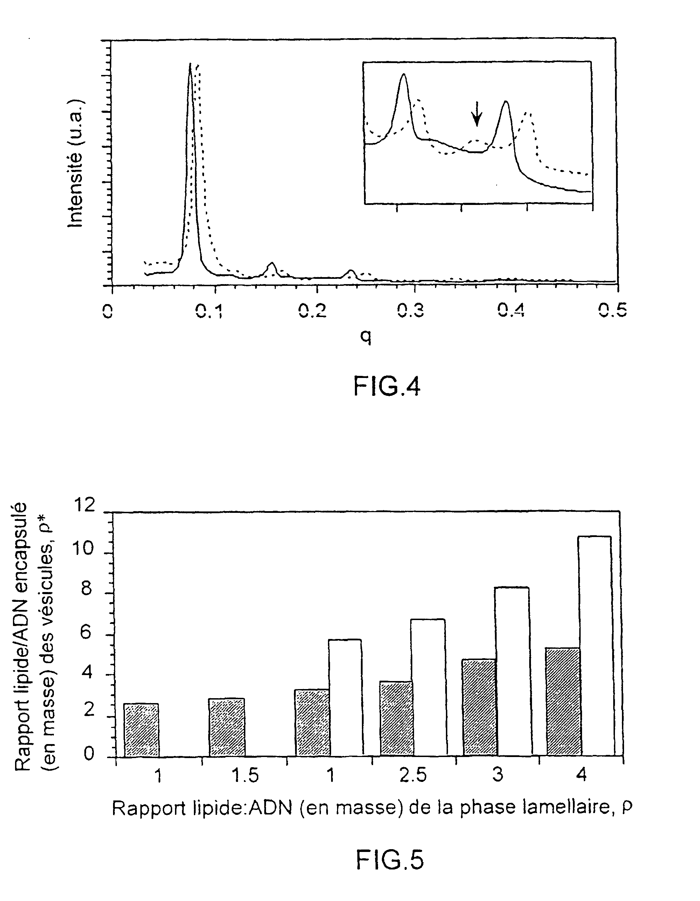

L'intégrité de la phase $L_\alpha^c$ après dispersion sous forme des vésicules a été également vérifiée par diffraction de rayons X. Les résultats apparaissent sur la figure 4. On peut noter que le pic de corrélation entre brins d'ADN est encore observable. La distance de répétition des bicouches lipidiques et de l'ADN est modifiée en fonction de la pression osmotique du milieu dispersant. Ceci démontre clairement que l'arrangement moléculaire est conservé même lors de la dispersion.

L'évolution du rapport massique lipide/ADN encapsulé dans les vésicules selon l'invention dispersées en milieu physiologique, $\rho*$, en fonction du rapport massique lipide/ADN de la phase $L_\alpha^c$ avant dispersion. $\rho$ . pour deux types d'ADN est représentée sur la figure 5. La représentation du pouvoir d'encapsulation par $\rho*$ plutôt que par le pourcentage de taux d'encapsulation est plus astucieuse car ceci correspond directement à la quantité d'ADN dans le vecteur. Pour l'ADN court ($\approx$ 150 p.b.) ainsi que pour le plasmide (5581 p.b.). $\rho*$ diminue (la quantité d'ADN dans le vecteur augmente) avec la diminution de $\rho$ de la phase $L_\alpha^c$. Le pouvoir d'encapsulation des vésicules de l'invention à base de lipide neutre est moindre pour le grand plasmide que pour l'ADN court. mais on atteint néanmoins des $\rho*$ de l'ordre de 6, donc l'encapsulation de quantités importantes de plasmide.

Les applications de cette technique sont très importantes. dans le domaine de la vectorisation de gènes pour la thérapie génique. En effet, le fait de pouvoir incorporer une grande quantité de macromolécules nucléotidiques avec un très bon rendement d'encapsulation est une condition importante de la réussite de telles approches. Mais, de plus, le fait de ne pas avoir à utiliser de lipides cationiques permet l'utilisation de tels systèmes *in vivo*, où les cationiques sont rapidement captés par les protéines et donc inefficaces. De plus les lipides neutres ont une cytotoxicité beaucoup plus faible que les lipides cationiques.

Ces considérations de capture par les protéines circulantes, d'adhésion aux parois cellulaires, et de cytotoxicité sont évidemment indépendantes de la nature de la macromolécule incorporée dans la vésicule.

### Exemples :

*Produit :*

Les matières premières utilisées dans les exemples sont les suivantes :

| | |
|---|---|
| 1,2-di-acyl-glycéro-phosphatidylcholine de soja | (Avanti Polar Lipid), |
| alcool laurique éthoxylé à 4 oxydes d'éthylène | (C12E4) (SEPPIC), |
| 1-monooleyl-*rac*-glycerol (monooléine) | (Sigma), |
| iodure de YOYO-1 | (Molecular Probes), |
| DNase type 1 | (Amersham France), |
| ADN hautement polymérisé de thymus de veau | (Sigma), |
| plasmide pCMV LUCIFERASI | (Qiagen GmbH). |

L'ADN de thymus de veau à été traité aux ultrasons pour obtenir des fragments double brins de la longueur de persistance de l'ADN. La taille de ces fragments a été vérifiée voisine de 150 paires de base (pb) par électrophorèse sur gel d'agarose.

Dans tous les exemples. les pourcentages sont en masse.

*Mélange des composants amphiphiles :*

Les différentes phases lamellaires ont été préparées comme suit :

Afin de simplifier la préparation. il est possible de préparer préalablement un mélange stock des différents tensioactifs utilisés dans la formulation. Ce mélange stock peut être préparé directement, soit à température ambiante, soit pour accélérer le mélange en chauffant légèrement. Il est aussi possible de dissoudre les différents tensioactifs dans un solvant (alcool et/ou solvant chloré, par exemple) pour les mélanger dans les bonnes proportions, puis d'évaporer le solvant sous vide afin d'obtenir le mélange stock des tensioactifs. Cette méthode permet aussi de filtrer la solution pour la stériliser si nécessaire.

Le mélange stock peut être conservé à -18°C pour une utilisation ultérieure.

*Préparation des phases lamellaires comtenant l'ADN :*

La quantité d'ADN pour obtenir le rapport massique lipide/ADN souhaite est ajoutée au mélange sec et homogène des lipides. L'ajout se fait le plus simplement en ajoutant la solution aqueuse d'ADN selon la quantité désirée. Le système est mélangé et laissé au repos pour permettre son homogénisation. Le temps nécessaire pour atteindre l'équilibre dépend de l'hydratation et du contenu en ADN du système (quelques jours en moyenne). La phase lamellaire homogène peut ensuite être stockée dans le noir à -20°C pendant des mois sans qu'elle perde ses caractéristiques et sans qu'il y ait dégradation chimique des constituants.

*Préparation des vésicules à partir de la phase* $L_\alpha^c$ *:*

La dispersion de la phase lamellaire dans un tampon à l'osmolarité désirée conduit à la phase de vésicules. Une agitation douce pendant quelques heures permet de disperser facilement les vésicules. L'homogénéité de la dispersion est contrôlée de manière ponctuelle par microscopie en contraste de phase.

Si l'on veut obtenir une meilleure homogénéité de taille. on peut aussi procéder par cisaillement de la phase structurée entre deux lames de verre propres. Le contrôle du cisaillement se fait par microscopie optique entre polariseurs croisés.

Dans le cas de la phase $L_\alpha^c$ contenant le plasmide. l'intégrité du plasmide après cisaillement et dispersion a été vérifiée par électrophorèse sur gel d'agarose.

*Mesure des pressions osmotiques :*

La pression osmotique à l'intérieur des vésicules contenant de l'ADN a été estimée à partir des mesures de la pression osmotique des solutions aqueuses d'ADN à 150 pb sur un osmomètre clinique de type 13 DR-Autocal. La pression osmotique de la solution aqueuse d'ADN évolue d'une façon non linéaire avec ia concentration d'ADN, comportement classique pour des macromolécules. Les estimations montrent que la pression osmotique de la phase $L_\alpha^c$ à $\rho$ = 2 et 50% d'eau (en masse) est de l'ordre de 500 milliosmomol ce qui correspond à une salinité d'environ 0.28 M. La pression osmotique des différents tampons utilisés a été ajustée en ajoutant du NaCl et vérifiée par mesure expérimentale.

*Protection de l'ADN dans les vésicules contre l'action de la DNase :*

Des vésicules contenant de l'ADN ainsi que l'ADN témoin non encapsulé ont été incubés avec de la DNase de type I en présence de $Mg^{2+}$. La DNase de type 1 est une endonucléase qui catalyse la dégradation des simples et doubles brins d'ADN et produit des oligonucléotides 5'-P terminaux. Elle nécessite la présence de cations divalents pour fonctionner. La dégradation par la DNase a été arrêtée à différents temps par l'ajout d'EDTA et l'ADN analysé par électrophorèse sur gel. Même longtemps après que l'ADN témoin a été détruit complètement, l'ADN encapsulé ne montre aucun signe de dégradation.

*Diffraction des rayons X :*

Pour la diffraction des rayons X, les échantillons sont transférés dans des capillaires de verre de 1 mm ou dans des supports plats (épaisseur traversée 1 mm) et scellés. Les expériences de diffraction de rayons X ont été faites soit sur un génerateur à anode tournante (Cu K$\alpha$ $\lambda$=1.5405Å) soit sur un synchrotron ($\lambda$=1.0A beamline ID2, ESRF, Grenoble) avec un détecteur 2D et des distances échantillon - détecteur variables (500 mm à 1000 mm).

Après regroupement les positions exactes des pics ainsi que leur intensité ont été obtenues par paramétrage gaussien des pics après soustraction du fond. Dans les cas où plusieurs pics par phase ont été obtenus, les distances de répétition, *d*, ont été déterminées par un paramétrage par les moindres carrés de la pente de $(h^2 + k^2 + l^2)^{1/2}$ pour les indices *hkl* (avec *l* et *k*=0 pour une phase lamellairer en fonction des espacements réciproques.

Les intensités. correspondant au carré de facteurs de structure. ont été corrigées pour le facteur de Lorentz et soumises à la normalisation de Blaurock. Les signes des facteurs de structure ont été obtenus par la méthode classique du gontlement. Les profils de densité électronique de la phase $L_\alpha^c$ ont été ensuite obtenus par transformation de Fourier inverse des facteurs de structure (voir par exemple : Franks,N.P. & Levine. W.R.. 1981, dans Membrane Spectroscopy (Springer Verlag, Heidelberg, 437-487).

Des expériences de diffraction de rayons X ont été faites sur des phases lamellaires contenant entre 25 et 70 % d'eau (en masse), c'est à dire dans la région des phases concentrées. avec ρ entre ∞ et 1 et sur des mélanges PC/C12E4 et PC/monooléine (95:5, en masse). Le choix du cotensioactif ne semble avoir aucune influence sur le diagramme de phases ternaire du système ADN/lipide neutre/eau. Par contre. les distances de répétition sont légèrement plus importantes pour les système contenant du C12E4 et la limite de gonflement est légèrement décalée vers les plus fortes concentrations en eau. L'ADN utilisé pour établir le diagramme de phases est préalablement traité aux ultrasons et d'une longueur d'environ 500Å ($\cong$150 pb).

Optiquement, on observe un excès d'eau pour la phase lamellaire a partir de 50% d'eau. Dans le cas des échantillons contenant de l'ADN, l'excès d'eau est observé entre 55 et 60% en fonction du contenu en ADN. La détermination partielle du diagramme de phases par diffraction des rayons X permet de distinguer de plus trois régimes : *(i)* les faibles concentrations en ADN (grand ρ) où les *d* sont plus faibles que celle de la phase lamellaire sans ADN (voir figure 1B), *(ii)* les concentrations intermédiaires (moyen ρ) où l'on observe la coexistence de deux phases lamellaires à l'équilibre (voir figure 1C) et *(iii)* les fortes concentrations d'ADN (petit p) où l'on détecte une seule phase lamellaire avant un d plus grand que celui de la phase lamellaire en absence d'ADN (voir figure 1D). La diminution de *d* de quelques Å dans le cas de faible contenu en ADN *(i)* peut s'expliquer uniquement si l'on considère que, au moins une partie de l'ADN n'est pas incorporée dans la phase lamellaire et induit une déshydratation partielle de cette dernière. Pour les valeurs moyennes de ρ *(ii)*, la présence d'un biphasique lamellaire-lamellaire ressemble à ce qui a été observé dans le cas des systèmes mixtes tensioactif/polymère/eau (Nallet, F. *et al.*, 1994, J. Phys. II France, 4,1477-1499). L'ADN se trouve essentiellement dans la phase à grand *d* (environ 110 à 130Å), la phase à faible *d* (autour de 50Å) est dépourvue d'ADN et légèrement déshydratée. Pour ρ $\leq$ 6. *(iii)*, la seule phase lamellaire présente est caractérisée par des *d* augmentés de 15 à 20Å par rapport à la phase lamellaire en absence d'ADN. Ceci démontre clairement que l'ADN est confiné entre les lamelles lipidiques. Contrairement au système lipide cationique/ADN le confinement ici est stérique puisqu'il n'y a pas d'interactions électrostatiques.

En plus des pics de la phase lamellaire on observe un pic large situé entre q $\cong$ 0,15 et 0,2 en fonction de la composition de la phase lamellaire (voir insert de la figure 1D). Ce pic n'existe pas pour des échantillons à ρ > 6 et a été attribué à la corrélation entre brins d'ADN comme il a été observé pour les systèmes lipides cationiques/ADN (J. O. Räder, *et al,* Science, **275** p 810 1997 ; T. Salditt *et al*, Physical Review Letters, **79** p 2582 1997). La distance entre brins d'ADN varie en effet avec ρ entre $\approx$30 et 42Å à hydratation constante. Ces distances sont caractéristiques d'une organisation nématique de l'ADN dans l'eau (Durand, D. *et al.*, 1992, J. Phys. II France 2, 1769-1783). Nous avons donc une phase lamellaire $L_\alpha^c$ où l'ADN est intercalé entre les bicouches smectiques et organisé en nématique-2D (voir aussi figure 1B). On observe également que la distance entre brins d'ADN diminue avec la diminution de l'hydratation à ρ constant. Ceci est dû à la restriction du wobbling (des oscillations) des double brins d'ADN quand l'espace interstitiel diminue.

La diffraction de rayons X sur des vésicules contenant l'ADN encapsulé et dispersées dans du tampon a été également réalisée. Pour ceci les vésicules ont été dispersées dans un large excès de tampon et ensuite concentrées. Cette étape de dilution suivie par la concentration élimine la quasi totalité d'ADN non encapsulé. Des résultats obtenus avec ces préparations sont montrés sur la figure 4. Le profil de diffraction est caractéristique d'une phase lamellaire et démontre le caractère multilamellaire des vésicules. La distance inter lamellaire d dépend, comme attendu de la pression osmotique du tampon dispersant et augmente de 15 à 20Å par rapport aux vésicules ne contenant pas d'ADN. De plus, on détecte facilement la présence d'un pic de corrélation entre brins d'ADN qui se déplace en fonction de la quantité d'ADN dans les vésicules et en fonction de la pression osmotique du milieu dispersant. Tout ceci est en parfait accord avec ce qui était trouvé pour les phases concentrées et prouve donc que la structure en phase $L_\alpha^c$ est préservée dans les vésicules.

Des expériences de diffraction des rayons X ont aussi été réalisées dans le but d'étudier le passage d'un milieu dispersant hyperosmotique à un milieu hypoosmotique sur des vésicules contenant de l'ADN (voir plus loin). Dans ce cas les vésicules ont été dispersées dans un large excès de solvant à 900 mOsm (hyperosmotique). Ensuite nous avons procédé à un échange du solvant en passant à 300 mOsm (milieu physiologique; hypoosmotique pour la phase $L_\alpha^c$). On constate que la structure lamellaire reste intacte même après passage dans le milieu hypoosmotique. Le *d* passe d'environ 78Å à 900 mOsm à environ 88Å à 300 mOsm, c'est à dire les d restent largement plus grands que dans le cas des vésicules ne contenant pas d'ADN ($\cong$63Å).

On a de plus reconstruit des profils de densité électronique de la phase $L_\alpha^c$ à partir des intensités des pics de Bragg de l'arrangement smectique. Le profil présenté dans la figure 2A montre une bosse de densité électronique importante à environ 40Å du centre de la bicouche lipidique et est cohérent avec la présence d'ADN dans la partie aqueuse de la phase lamellaire.

*Détermination de taux d'encapsulation d'ADN dans les vésicules :*

Le taux d'encapsulation a été déterminé par spectrofluorimétrie en utilisant comme fluorophore le YOYO ($\lambda_{ex}$ = 491 nm, $\lambda_{em}$ = 509 nm). Sauf indication contraire les résultats cités ci-dessous ont été obtenus avec l'ADN traité aux ultrasons de ≈150 pb.

L'ADN non encapsulé est mesuré par l'ajout de YOYO aux dispersions aqueuses des vésicules ($I_D$). L'ADN total est dosé après destruction des vésicules par le Triton ($I_T$). Le taux d'encapsulation s'obtient par: % = 100·($I_T$-$I_D$)/($I_T$-$I_0$) avec $I_0$ = intensité du YOYO seul. La conversion en rapport massique lipide/ADN encapsulé s'obtient par : $\rho^*$ = 100·$\rho$/taux d'encapsulation.

Des mesures du taux d'encapsulation d'ADN dans les vésicules en fonction de la pression osmotique du tampon ont été réalisées. Pour ceci les vésicules ont été préparées à partir d'une phase $L_\alpha^c$ à $\rho$ = 2 et à 55 % d'hydratation. La pression osmotique à l'intérieur des vésicules a été estimée de l'ordre de 500 mÖsm. Sur la figure 3, on voit que le taux d'encapsulation augmente progressivement jusqu'à des pressions osmotiques d'environ 600 mOsm et reste constant pour des pressions osmotiques plus fortes démontrant l'importance de la pression osmotique pour l'optimisation de taux d'encapsulation. Ces résultats suggèrent également que la pression osmotique à l'intérieur des vésicules est probablement légèrement plus forte que les estimations obtenues à partir des solutions aqueuses d'ADN. Ceci est probablement dû à la présence de sels dans le phospholipide d'origine naturelle.

On notera néanmoins que l'optimum de taux d'encapsulation de l'ADN se situe à des pressions osmotiques supérieures à celles du milieu physiologique (300 mOsm). Nous avons donc ensuite étudié la dilution des vésicules (phase $L_\alpha^c$ : $\rho$ = 2, 55 % d'eau) préparées dans un milieu hyperosmotique (900 mOsm) dans un milieu à 300 mOsm et hypoosmotique pour la phase $L_\alpha^c$. Dans ce cas on observe que d'une manière totalement surprenante le taux d'encapsulation ne descend pas à celui des vésicules préparées à 300 mOsm mais reste identique à celui obtenu à 900 mOsm. Il est donc possible de préparer des vésicules contenant l'ADN à forte pression osmotique et de les diluer dans un milieu physiologique au moment de l'application sans qu'il y ait perte d'ADN encapsulé.

L'étude de la stabilité de ces vecteurs d'ADN en fonction du temps montre que la meilleure stabilité est obtenue dans un milieu hyperosmotique où l'on n'observe qu'une légère baisse du taux d'encapsulation de ≅70 à ≅60% au cours de 30 jours.

Pour l'étude d'optimisation en fonction de la composition de la phase $L_\alpha^c$ servant à la fabrication des vésicules, les vecteurs ont été préparés dans un tampon hyperosmotique et le taux d'encapsulation mesuré après leur dilution dans un tampon physiologique.

En faisant varier le contenu en eau de la phase $L_\alpha^c$ entre 40 et 70 % à $\rho$ constant et égal à 2, on trouve un maximum du taux d'encapsulation à une hydratation correspondant à la limite de gonflement de la phase $L_\alpha^c$ (55 % d'eau). Pour les hydratations au-dessus de 55%, la baisse de l'encapsulation s'explique par le fait que l'ADN, soluble dans le solvant, se répartit entre la phase $L_\alpha^c$ et l'excès de solvant et est donc de moins en moins présent à l'intérieur de la phase $L_\alpha^c$. En-dessous de 55%, la diminution du taux d'encapsulation quand l'hydratation baisse résulte probablement de la faible hydratation et de l'augmentation de la viscosité qui ont gêné l'obtention des vésicules.

Conservant un taux d'hydratation de 50 % qui correspond à une encapsulation maximale, nous avons étudié l'influence du $\rho$ de la phase $L_\alpha^c$ sur le taux d'encapsulation. Les résultats montrent que d'une manière générale l'encapsulation est facilitée par l'abondance de lipides (70% pour p = 4 contre 45% pour $\rho$ = 1). Il est néanmoins important de réaliser que ce résultat en % d'ADN encapsulé ne donne aucune indication sur l'évolution de la quantité d'ADN encapsulée. Les résultats convertis en rapport massique lipide/ADN encapsulé $\rho^*$ sont représentés sur la figure 5 où l'on voit que, pour $\rho$=4, malgré un taux d'encapsulation élevé (70%), la quantité totale d'ADN dans les vésicules est inférieure à celle encapsulée pour $\rho$ = 1. Concernant l'optimisation de l'encapsulation, il apparaît donc deux points de vue : (i) soit on introduit une grande quantité d'ADN ($\rho$ faible), la quantité encapsulée est élevée mais on observe également beaucoup d'ADN dans le milieu extérieur, *(ii)* soit on introduit une faible quantité d'ADN (p élevé) qui sera en majorité encapsulée. mais on aura globalement moins d'ADN dans les vésicules.

L'influence du p de la phase lamellaire a été également étudiée pour les vésicules contenant du plasmide. Dans ce cas, les taux d'encapsulation restent à peu près constant autour de 40% entre $\rho$ = 4 et 2, c'est à dire plus faible que pour l'ADN court. La conversion en $\rho^*$ en fonction du p montre néanmoins que les deux systèmes évoluent d'une façon comparable (voir figure 5). Ce résultat permet de transposer au plasmide les conclusions obtenues à partir du système contenant l'ADN à 150 p.b.

## Revendications

1. Phase mixte structurée formée par l'incorporation de macromolécules biologiques arrangées selon une structure cristal-liquide dans une phase cristal-liquide d'amphiphiles, **caractérisée en ce que** ladite macromolécule biologique est présente dans ladite phase mixte structurée à raison d'au moins 5 % en poids par rapport aux composés amphiphiles constituant ladite phase cristal-liquide et **en ce que** l'une au moins des deux entités constituées par la macromolécule biologique d'une part, et l'ensemble des composés amphiphiles d'autre part, présente un caractère non chargé.

**2.** Phase mixte structurée selon la revendication 1, **caractérisée en ce que** ladite phase cristal-liquide d'amphiphiles est une phase cristal-liquide lamellaire.

**3.** Phase mixte structurée selon la revendication 2, **caractérisée en ce que** ladite macromolécule biologique présente un rayon de giration supérieur à la distance entre les lamelles amphiphiles de ladite phase cristal-liquide.

**4.** Phase mixte structurée selon l'une des revendications 1 à 3, **caractérisée en ce que** ladite macromolécule biologique est choisie dans le groupe constitué des polynucléotides, des protéines, des polysaccharides.

**5.** Phase mixte structurée selon l'une des revendications 1 à 4, **caractérisée en ce que** ladite macromolécule est un polynucléotide, de préférence l'ADN ou l'ARN.

**6.** Phase mixte structurée selon l'une des revendications 1 à 5, **caractérisée en ce que** lesdits composés amphiphiles constituant la phase structurée d'amphiphiles sont choisis dans le groupe constitué de :

- ◆ des glycérolipides, en particulier des glycérolipides phospholipidiques, hydrogénés ou non,
- ◆ des acides gras en $C_6$ à $C_{30}$, saturés ou mono- ou polyinsaturés, linéaires ou ramifiés, éthoxylés ou non, sous forme d'acide ou de sel d'un métal alcalin, alcalino-terreux ou d'une amine,
- ◆ des esters éthoxylés ou non, de saccharose, ou de sorbitol, ou de mannitol, ou de glycérol ou de polyglycérol contenant de 2 à 20 motifs glycérol ou de glycol de ces mêmes acides gras,
- ◆ des mono-, di- ou triglycérides ou des mélanges de glycérides de ces mêmes acides gras,
- ◆ des alcools gras en $C_6$ à $C_{30}$, saturés ou mono- ou polyinsaturés, linéaires ou ramifiés, éthoxylés ou non,
- ◆ du cholestérol et ses dérivés, en particulier les esters de cholestérol chargés ou neutres comme le sulfate de cholestérol,
- ◆ des autres dérivés à squelette stérol, en particulier ceux d'origine végétale tels que le sitostérol ou le sigmastérol,
- ◆ des éthers, éthoxylés ou non, de saccharose, ou de sorbitol, ou de mannitol, ou de glycérol ou de polyglycérol contenant de 2 à 20 motifs glycérol ou de glycol et de ces mêmes alcools gras,
- ◆ des huiles végétales polyéthoxylées, hydrogénées ou non hydrogénées,
- ◆ des polymères séquences de polyoxyéthylène et de polyoxypropylène (poloxamères),
- ◆ de l'hydroxystéarate de polyéthylèneglycol,
- ◆ des sphingolipides, et des dérivés de la sphingosine,
- ◆ des polyalkylglucosides,
- ◆ des céramides,
- ◆ des copolymères de polyéthylèneglycol et d'alkylglycol
- ◆ des copolymères di- ou tribloc d'éthers de polyéthylèneglycol et de polyalkylèneglycol.

**7.** Phase mixte structurée selon l'une des revendications 1 à 6, **caractérisée en ce qu'**elle contient en outre au moins un additif destiné à conférer des propriétés spécifiques, par exemple un additif destiné à conférer une propriété d'adhésion de surface à ladite phase mixte structurée.

**8.** Phase mixte structurée selon la revendication 7, **caractérisée en ce que** ledit additif est un tensioactif ou un polymère.

**9.** Procédé de préparation d'une phase mixte structurée selon l'une des revendications 1 à 8, **caractérisé en ce qu'**il comprend les étapes de :

- préparation d'un mélange homogène contenant les composés amphiphiles et la macromolécule, ladite macromolécule étant présente dans des proportions pondérales d'au moins 5 % par rapport auxdits composés amphiphiles,
- d'incorporation d'une quantité de milieu aqueux ou polaire adéquate pour former une phase cristal-liquide d'amphiphiles avec lesdits composés amphiphiles.

**10.** Procédé de préparation de vésicules, **caractérisé en ce qu'**il comprend une étape de dispersion d'une phase mixte structurée telle que définie dans l'une des revendications 1 à 8 ou obtenue selon le procédé de la revendication 9.

**11.** Vésicules susceptibles d'être obtenues par dispersion d'une phase mixte structurée telle que définie dans l'une des revendications 1 à 8 ou obtenue selon le procédé de la revendication 9.

**12.** Dispersion de vésicules selon la revendication 11, **caractérisée en ce que** le milieu dans lequel lesdites vésicules se trouvent dispersées présente une osmolarité différente de celle du milieu interne de la phase mixte structurée d'amphiphiles.

**13.** Procédé de préparation d'une dispersion selon la revendication 12, **caractérisé en ce qu'**il comprend les étapes de :

- dispersion dans un milieu de même osmolarité que le milieu interne
- modification de l'osmolarité du milieu externe par ajout de sel ou d'eau ou par dialyse afin d'atteindre l'osmolarité souhaitée.

**Patentansprüche**

**1.** Strukturierte Mischphase, die durch Aufnahme von biologischen Makromolekülen, welche in Flüssigkristallstruktur angeordnet sind, in eine Flüssigkristallphase aus Amphiphilen gebildet wird, **dadurch gekennzeichnet, daß** das biologische Makromolekül in der strukturierten Mischphase zu einem Gehalt von mindestens 5 Gew.% bezogen auf die die Kristallphase bildenden Amphiphile vorliegt, und mindestens eine der zwei Einheiten, die durch das biologische Makromolekül einerseits und die Gesamtheit der Amphiphilen andererseits begründet werden, einen nicht-geladenen Charakter aufweist.

**2.** Strukturierte Mischphase nach Anspruch 1, **dadurch gekennzeichnet, daß** die Flüssigkristallphase aus Amphiphilen eine lamellare Flüssigkristallphase ist.

**3.** Strukturierte Mischphase nach Anspruch 2, **dadurch gekennzeichnet, daß** das biologische Makromolekül einen Drehradius aufweist, der größer ist als der Abstand zwischen den amphiphilen Lamellen der Flüssigkristallphase.

**4.** Strukturierte Mischphase nach einem der Ansprüche 1 bis 3, **dadurch gekennzeichnet, daß** das biologische Makromolekül ausgewählt ist aus der Gruppe bestehend aus Polynukleotiden, Proteinen, Polysacchariden.

**5.** Strukturierte Mischphase nach einem der Ansprüche 1 bis 4, **dadurch gekennzeichnet, daß** das Makromolekül ein Polynukleotid, vorzugsweise DNA oder RNA ist.

**6.** Strukturierte Mischphase nach einem der Ansprüche 1 bis 5, **dadurch gekennzeichnet, daß** die die strukturierte Phase aus Amphiphilen bildenden amphiphilen Verbindungen ausgewählt sind aus der Gruppe bestehend aus:

- Glycerolipiden, insbesondere phospholipidischen Glycerolipiden, hydriert oder nicht hydriert,
- Fettsäuren von $C_6$ bis $C_{30}$, gesättigt oder einfach oder mehrfach ungesättigt, linear oder verzweigt, ethoxyliert oder nicht ethoxyliert, in Form einer Säure oder eines Salzes eines Alkalimetalls, Erdalkalimetalls oder eines Amins,
- ethoxylierten oder nicht ethoxylierten Estern von Saccharose, Sorbitol, Mannitol, Glycerin, Polyglycerin, das 2-20 Einheiten Glycerin enthält, oder Glykol mit diesen Fettsäuren,
- Mono-, Di- oder Triglyceriden oder Gemischen von Glyceriden dieser Fettsäuren,
- Fettalkoholen von $C_6$ bis $C_{30}$, gesättigt oder einfach oder mehrfach ungesättigt, linear oder verzweigt, ethoxyliert oder nicht ethoxyliert,
- Cholesterin und dessen Derivaten, insbesondere geladenen oder neutralen Cholesterinestern, wie etwa Cholesterinsulfat,
- weiteren Derivaten mit Sterolskelett, insbesondere von pflanzlicher Herkunft, wie etwa Sitosterol oder Stigmasterol,
- ethoxylierten oder nicht ethoxylierten Ethern von Saccharose, Sorbitol, Mannitol, Glycerin, Polyglycerin, das 2-20 Einheiten Glycerin enthält, oder Glykol mit diesen Fettalkoholen,
- pflanzlichen Ölen, polyethoxyliert, hydriert oder nicht hydriert,
- Blockpolymeren aus Polyoxyethylen und Polyoxypropylen (Poloxamere),
- Polyethylenglykolhydroxystearat,
- Sphingolipiden und Sphingosinderivaten,
- Polyalkylglucosiden,

- Ceramiden,
- Copolymeren von Polyethylenglykol und Alkylglykol,
- Di- oder Triblock Copolymeren aus Ethern von Polyethylenglykol und Polyalkylenglykol.

**7.** Strukturierte Mischphase nach einem der Ansprüche 1 bis 6, **dadurch gekennzeichnet, daß** sie ferner mindestens einen Zusatzstoff enthält, der dazu vorgesehen ist, spezifische Eigenschaften zu vermitteln, beispielsweise einen Zusatzstoff, der dazu vorgesehen ist, der strukturierten Mischphase eine Oberflächenadhäsionseigenschaft zu verleihen.

**8.** Strukturierte Mischphase nach Anspruch 7, **dadurch gekennzeichnet, daß** der Zusatzstoff ein Tensid oder ein Polymer ist.

**9.** Verfahren zur Herstellung einer strukturierten Mischphase nach einem der Ansprüche 1 bis 8, **dadurch gekennzeichnet, daß** es die Schritte:

- Herstellung eines homogenen Gemischs, welches die amphiphilen Verbindungen und das Makromolekül enthält, wobei das Makromolekül zu mindestens 5 % Gewichtanteilen bezogen auf die amphiphilen Verbindungen vorliegt,
- Aufnahme einer angemessenen Menge von wäßrigem oder polarem Medium zur Bildung einer Flüssigkristallphase aus Amphiphilen mit den amphiphilen Verbindungen,

umfaßt.

**10.** Verfahren zur Herstellung von Vesikeln, **dadurch gekennzeichnet, daß** es einen Schritt der Dispersion einer strukturierten Mischphase, wie in einem der Ansprüche 1 bis 8 beschrieben oder nach dem Verfahren aus Anspruch 9 erhalten, umfaßt.

**11.** Vesikel erhältlich durch Dispersion einer strukturellen Mischphase, wie in einem der Ansprüche 1 bis 8 beschrieben oder nach dem Verfahren nach Anspruch 9 erhalten.

**12.** Vesikeldispersion nach Anspruch 11, **dadurch gekennzeichnet, daß** das Medium, in dem die Vesikel dispergiert sind, eine Osmolarität aufweist, die sich von jener des internen Mediums der strukturierten Mischphase aus Amphiphilen unterscheidet.

**13.** Verfahren zur Herstellung einer Dispersion nach Anspruch 12, **dadurch gekennzeichnet, daß** es die Schritte:

- Dispersion in einem Medium von gleicher Osmolarität wie das interne Medium,
- Veränderung der Osmolarität des externen Mediums durch Zugabe von Salz oder Wasser oder durch Dialyse zum Erhalt der gewünschten Osmolarität,

umfaßt.

**Claims**

**1.** A structured mixed phase formed by the incorporation of biological macromolecules having a liquid-crystal structural arrangement into a liquid-crystal phase of amphiphiles, wherein said biological macromolecule is present in said structured mixed phase in an amount of at least 5% by weight, based on the amphiphilic compounds constituting said liquid-crystal phase, and wherein at least one of the two entities consisting of the biological macromolecule, on the one hand, and all the amphiphilic compounds, on the other, is of an uncharged nature.

**2.** A structured mixed phase according to claim 1 wherein said liquid-crystal phase of amphiphiles is a lamellar liquid-crystal phase.

**3.** A structured mixed phase according to claim 2 wherein the radius of gyration of said biological macromolecule is greater than the distance between the amphiphilic lamellae of said liquid-crystal phase.

**4.** A structured mixed phase according to one of claims 1 to 3 wherein said biological macromolecule is selected from the group consisting of polynucleotides, proteins and polysaccharides.

5. A structured mixed phase according to one of claims 1 to 4 wherein said macromolecule is a polynucleotide, preferably DNA or RNA.

6. A structured mixed phase according to one of claims 1 to 5 wherein said amphiphilic compounds constituting the structured phase of amphiphiles are selected from the group consisting of :

- ♦ glycerolipids, particularly hydrogenated or non-hydrogenated phospholipidic glycerolipids,
- ♦ ethoxylated or non-ethoxylated, linear or branched, saturated or mono- or polyunsaturated C6 to C30 fatty acids in the form of the acid or an alkali metal, alkaline earth metal or amine salt,
- ♦ ethoxylated or non-ethoxylated esters of sucrose, sorbitol, mannitol, glycerol, polyglycerol containing from 2 to 20 glycerol units, or glycol with the above fatty acids,
- ♦ mono-, di- or triglycerides or mixtures of glycerides of the above fatty acids,
- ♦ ethoxylated or non-ethoxylated, linear or branched, saturated or mono- or polyunsaturated C6 to C30 fatty alcohols,
- ♦ cholesterol and derivatives thereof, particularly charged or neutral cholesterol esters such as cholesterol sulfate,
- ♦ other derivatives with a sterol skeleton, particularly those of vegetable origin such as sitosterol or sigmasterol,
- ♦ ethoxylated or non-ethoxylated ethers of sucrose, sorbitol, mannitol, glycerol, polyglycerol containing from 2 to 20 glycerol units, or glycol with the above fatty alcohols,
- ♦ hydrogenated or non-hydrogenated, polyethoxylated vegetable oils,
- ♦ polyoxyethylene/polyoxypropylene block polymers (poloxamers),
- ♦ polyethylene glycol hydroxystearate,
- ♦ sphingolipids and sphingosine derivatives,
- ♦ polyalkyl glucosides,
- ♦ ceramides,
- ♦ polyethylene glycol/alkyl glycol copolymers,
- ♦ polyethylene glycol/polyalkylene glycol ether di-block or tri-block copolymers.

7. A structured mixed phase according to one of claims 1 to 6 which also contains at least one additive for conferring specific properties, for example an additive for conferring a surface adhesion property on said structured mixed phase.

8. A structured mixed phase according to claim 7 wherein said additive is a surfactant or a polymer.

9. A process for the preparation of a structured mixed phase according to one of claims 1 to 8 which comprises the following steps:

- preparation of a homogeneous mixture containing the amphiphilic compounds and the macromolecule, said macromolecule being present in proportions of at least 5% by weight, based on said amphiphilic compounds, and
- incorporation of an appropriate amount of aqueous or polar medium to form a liquid-crystal phase of amphiphiles with said amphiphilic compounds.

10. A process for the preparation of vesicles which comprises a step for dispersion of a structured mixed phase as defined in one of claims 1 to 8 or obtained by the process of claim 9.

11. Vesicles obtainable by the dispersion of a structured mixed phase as defined in one of claims 1 to 8 or obtained by the process of claim 9.

12. A dispersion of vesicles according to claim 11 wherein the medium in which said vesicles are dispersed has a different osmolarity from that of the internal medium of the structured mixed phase of amphiphiles.

13. A process for the preparation of a dispersion according to claim 12 which comprises the following steps:

- dispersion in a medium of the same osmolarity as the internal medium, and
- modification of the osmolarity of the external medium by the addition of salt or water or by dialysis in order to attain the desired osmolarity.

FIG.1A

FIG.1B

FIG.1C

FIG.1D

Densité éléctronique (u.a.)

Distance du centre de la bicouche (Å)

FIG.2A

FIG.2B

Taux d'encapsulation d'ADN (%)

mOsm

FIG.3

FIG.4

FIG.5